Europäisches Patentamt

European Patent Office

Office européen des brevets

(19)

(11) Numéro de publication: **0 536 214 B1**

(12) **FASCICULE DE BREVET EUROPEEN**

(45) Date de publication du fascicule du brevet: **22.11.95** (51) Int. Cl.6: **C12Q 1/68**

(21) Numéro de dépôt: **91911799.4**

(22) Date de dépôt: **27.06.91**

(86) Numéro de dépôt internationale :
**PCT/FR91/00513**

(87) Numéro de publication internationale :
**WO 92/00384 (09.01.92 92/02)**

Le dossier contient des informations techniques
présentées postérieurement au dépôt de la
demande et ne figurant pas dans le présent
fascicule.

(54) **PERFECTIONNEMENTS A UN PROCEDE D'AMPLIFICATION ENZYMATIOUE IN VITRO.**

(30) Priorité: **29.06.90 FR 9008247**

(43) Date de publication de la demande:
**14.04.93 Bulletin 93/15**

(45) Mention de la délivrance du brevet:
**22.11.95 Bulletin 95/47**

(84) Etats contractants désignés:
**AT BE CH DE DK ES FR GB GR IT LI LU NL SE**

(56) Documents cités:
**EP-A- 0 401 037     EP-A- 0 407 291**
**EP-A- 0 415 755     WO-A-89/09284**
**WO-A-89/11548     WO-A-90/01563**
**WO-A-90/06376     WO-A-90/12115**

**NATURE vol. 343, 04 janvier 1990; G. SARKAR
et al., p. 27 &NUM;**

(73) Titulaire: **GENSET**
**1, rue Robert et Sonia Delaunay**
**F-75011 Paris (FR)**

(72) Inventeur: **BRANDYS, Pascal**
**14, rue Gardenat-Lapostol**
**F-92150 Suresnes (FR)**
Inventeur: **d'AURIOL, Luc**
**27, rue de Mouzaia**
**F-75019 Paris (FR)**

(74) Mandataire: **Warcoin, Jacques et al**
**Cabinet Régimbeau,**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

NUCLEIC ACIDS RESEARCH vol. 18, no. 22, 1990; Y. JINNO et al., p. 6739 &NUM;

ucleic Acids Research, vol. 17, no. 14, 25 juillet 1989, IRL Press, R.G. Higuchi et al.: "Production of single-stranded DNA templates by exonuclease digestion following the polymerase chain reaction", page 5865, voir lignes 9-18

Nature, vol. 343, 4 janvier 1990, G. Sarkar et al.: "Shedding light on PCR contamination", page 27, voir l'article en entier

Proceedings of the National Academy of Sciences of the USA, vol. 82, février

1985, G.B. Dreyer et al.: "Sequence-specific cleavage of single-stranded DNA: Oligo-deoxynucleotide-EDTA-Fe(II)", pages 968-972, voir le résumé

Nucleic Acids Research, vol. 18, no. 22, 1990, Y. Jinno et al: "Use of psoralen as extinguisher of contaminated DNA in PCR", page 6739, voir l'article en entier

Nucleic Acids Research, vol. 19, no. 1, G.D. Cimino et al.: "Post-PCR sterilization: a method to control carryover contamination for the polymerase chain reaction", pages 99-107, voir l'article en entier

**Description**

L'invention a pour objet un perfectionnement apporté à la technique d'amplification enzymatique in vitro, communément désignée sous l'appellation procédé PCR.

Les principes à la base de cette technique ont été déjà amplement décrits et sa mise en oeuvre a fait l'objet de dépôts de demandes de brevet. On peut citer à cet effet les publications européennes de brevet EP-A-201 184 et EP-A-200 362.

Pour la meilleure compréhension de l'invention, on rappellera ci-après les principaux éléments qui caractérisent le procédé PCR ainsi que les diverses applications auxquelles il peut donner lieu.

La technique PCR permet d'amplifier considérablement une séquence d'ADN spécifique, appelée séquence d'ADN cible, au sein d'un mélange d'ADN hétérogène.

Elle est basée sur le principe de la réplication de l'ADN par duplication enzymatique et l'utilisation d'amorces spécifiques encadrant la séquence d'ADN à amplifier, les extrémités 3' de ces amorces étant utilisées comme point de démarrage pour les enzymes de réplication de l'ADN, appelées ADN polymérases.

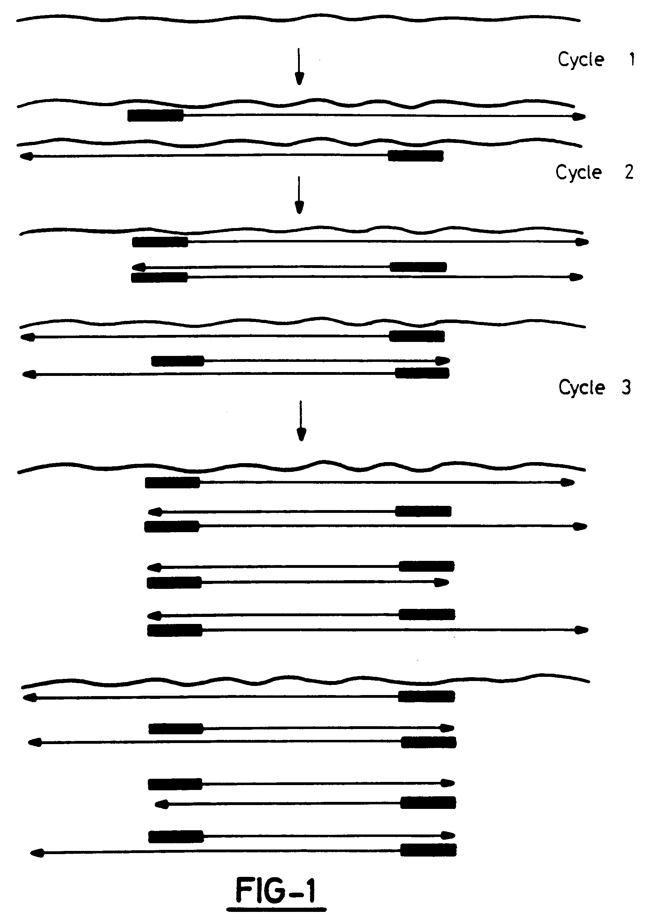

Le schéma de principe de cette technique est rappelé figure 1.

L'amplification de la séquence procède par cycles successifs. Chaque cycle comporte trois étapes : une première étape de dénaturation thermique de l'ADN, qui sépare l'ADN double brin en deux mono-brins, une seconde étape d'hybridation des amorces sur les séquences des mono-brins qui leur sont complémentaires, enfin une dernière étape d'élongation a partir des amorces à l'aide d'une ADN polymérase.

Pour mettre en oeuvre une amplification par PCR, on prépare un milieu réactionnel qui contient l'ADN hétérologue à analyser, les amorces, l'ADN polymérase. Ce milieu peut également contenir d'autres éléments tels qu'un tampon, des sels, des désoxynucléotides tri-phosphates.

Un des intérêts de cette technique est qu'il n'est pas toujours nécessaire de purifier l'ADN à analyser ; la technique peut être mise en oeuvre directement à partir d'un lysat cellulaire par exemple. En pratique, les échantillons d'ADN peuvent être préparés de façon très variée.

De façon non limitative, on peut citer des procédures de purification comportant par exemple des extractions au phénol ou par tout autre solvant, ou encore des passages dans des systèmes d'affinité. Dans le cas où la préparation ne comporte que des étapes de lyse de l'échantillon, la lyse peut être effectuée directement avant l'amplification.

Quelles que soient les procédures utilisées, elles conduisent à des molécules d'ADN dont les longueurs se trouvent aux environs de 0,5 à 20 kb alors que la longueur des séquences choisies comme cibles d'amplification est généralement de l'ordre de quelques centaines de nucléotides, soit environ 0,1 kb à 0,5 Kb. On se limite généralement à cet ordre de grandeur, pour des raisons d'efficacité de la technique. On peut toutefois rechercher dans certains cas l'amplification de segments d'ADN dont la longueur dépasse plusieurs centaines de nucléotides.

Pour préparer les amorces qui vont s'hybrider sur chaque monobrin après l'étape de dénaturation de l'ADN, on peut procéder de différentes façons. Généralement, on connait quelques séquences d'ADN bordant le fragment d'ADN à amplifier, on procède alors par synthèse des oligonucléotides amorces correspondant à ces séquences. Ces oligonucléotides peuvent, avant utilisation, être modifiés sans pour autant éliminer leur capacité à s'hybrider sur les séquences cibles ni leur capacité à servir de point de démarrage pour l'élongation. Par exemple, on peut ajouter à leurs extrémités 5' un site de restriction enzymatique, ce qui permet par la suite de cloner le fragment d'ADN amplifié après coupure par les enzymes de restriction correspondantes. On peut aussi introduire au niveau des extrémités 5' un promoteur, ce qui permet d'étudier par la suite les produits de transcription.

Le milieu étant ainsi préparé, le cycle réactionnel peut commencer. Les conditions opératoires des différentes étapes se distinguent essentiellement par la température à laquelle elles ont lieu. Les conditions de dénaturation correspondent généralement à une élévation de la température du milieu réactionnel au-dessus de 90°, l'hybridation a lieu généralement entre 50 et 60°C et l'élongation par l'ADN polymérase peut s'effectuer à des températures relativement élevées, de l'ordre de 0°C si l'on utilise une ADN polymérase stable à la chaleur. Dans ce cas, l'ensemble de cycles opératoires peut avoir lieu sans qu'il soit nécessaire de rajouter des enzymes en cours de cycle.

Après un premier cycle, une séquence cible présente en $x$ exemplaires dans l'échantillon à tester se trouvera dupliquée, c'est-à-dire présente à $2\,x$ exemplaires. Après $n$ cycles d'amplification, le nombre de séquences cibles sera multiplié par $2^n$, c'est-à-dire qu'une séquence cible présente initialement en $x$ exemplaires sera présente en $x.2^n$ exemplaires (voir figure 1). Même si les efficacités expérimentales ne sont pas de 100 % à chaque cycle, cette croissance exponentielle théorique du facteur d'amplification de la séquence cible donne une bonne illustration de la puissance de cette procédure. Les procédures de PCR couramment utilisées font état de 20, 30, voire 40 cycles d'amplification, ce qui correspond à des facteurs de multiplication théoriques de $2^{20}$ de $2^{30}$ ou de $2^{40}$ de la quantité d'échantillon initial.

La puissance de cette technique en fait un outil extrêmement précieux en recherche fondamentale ou clinique. Elle trouve des applications pratiques immédiates à des fins de pronostic ou diagnostic, pár exemple pour détecter la présence de séquences d'ADN d'intérêt médical correspondant à des microorganismes infectieux ou encore à des gènes cellulaires impliqués dans des pathologies diverses.

Toutefois, la puissance même de cette technique la rend extrêmement sensible à des risques de contamination des échantillons à analyser. L'une des conséquences les plus immédiates d'une contamination éventuelle est l'obtention de faux positifs.

Plus précisément, le contaminant majeur d'un échantillon négatif pouvant se répandre aisément dans un laboratoire pratiquant cette technologie, est le produit d'amplification lui-même tel qu'obtenu antérieurement au cours de l'analyse d'un autre échantillon en utilisant les mêmes amorces.

Ainsi, une fraction, aussi minime soit-elle, d'un produit d'amplification obtenu antérieurement, fera l'objet, dans un procédé ultérieur destiné à l'analyse d'une autre séquence cible, présente dans un échantillon initialement négatif pour la séquence cible du procédé antérieur, d'une amplification considérable et pourra donner lieu à un résultat positif pour la séquence cible antérieure à la place d'un résultat négatif.

La définition d'un produit d'amplification au sens de l'invention, sera mieux comprise en liaison avec la figure 1.

On peut voir que les élongations spécifiques effectuées par l'ADN polymérase aboutissent à la génération de produits qui présentent des structures moléculaires différentes. Les élongations effectuées sur les molécules cibles originelles de l'échantillon donnent naissance à des brins d'ADN commençant au niveau d'une amorce et dont la longueur est statistique, s'arrêtant à des distances variées de leur point d'initiation. A partir d'un brin d'ADN ainsi polymérisé, et dont la séquence commence exactement par l'amorce spécifique, un second cycle d'amplification générera une molécule d'ADN complémentaire, commençant au niveau de la seconde amorce et se terminant au plus long, après la duplication de l'amorce située du côté 5' du brin ainsi dupliqué. Ce nouveau brin d'ADN aura une longueur précisément égale à la distance séparant les extrémités 5' des deux amorces sur l'ADN cible. Un tel brin d'ADN, de longueur finie, et comportant à chacune de ses extrémités la séquence directe ou complémentaire des oligonucléotides amorces choisis pour l'amplification sera ultérieurement lui-même amplifié en donnant à chaque cycle un produit identique à lui-même. On peut calculer qu'en partant de $\underline{x}$ brins d'ADN soumis à $\underline{n}$ cycles d'amplification, on obtiendra $\underline{x}.2^n$ brins, dont $\underline{x}(n+1)$ auront une longueur variable et $\underline{x}.2^n\text{-}\underline{x}.(n+1)$ auront la longueur discrète du fragment délimité par les amorces d'amplification.

Le rapport, au bout des n cycles d'amplification entre la quantité de molécules de longueur variable et de longueur discrète sera donc de :

$$\frac{x.2^n\text{-}x.(n+1)}{x.(n+1)}$$

ce qui représente un excès considérable de molécules de longueur finie par rapport aux molécules de longueur variable. Ce sont ces molécules d'ADN de longueur finie constituées de la séquence d'ADN cible à analyser délimitée à chaque extrémité par les deux oligonucléotides amorces, que l'on appelle produits d'amplification. Elles contiennent en effet tous les éléments nécessaires pour une réamplification ultérieure.

Par contre, les molécules amplifiées de longueur variable ont en commun la caractéristique de commencer, du côté 5', par l'un des oligonucléotides amorces utilisés pour l'amplification.

Des considérations qui précèdent, il résulte que lorsqu'on initie une nouvelle réaction PCR à partir d'un échantillon d'ADN hétérologue, il est indispensable que le milieu soit dépourvu de toute contamination, venant soit des matériels, soit des instruments utilisés, et notamment due à des produits d'amplification issus d'un cycle PCR antérieur utilisant des oligonucléotides amorces identiques.

Idéalement, un milieu réactionnel non contaminé par des produits d'amplification antérieure ne comportera donc la séquence d'ADN cible à amplifier qu'au sein de molécules d'ADN de longueur variable, de l'ordre de 0,5 à 20 kb, comme indiqué précédemment, avec une représentation statistique de la localisation de la séquence cible sur une portion centrale ou distale des molécules d'ADN. La séquence d'ADN à amplifier sera donc comprise dans une molécule plus longue, entourée de part et d'autre de ses extrémités définies comme les régions d'hybridation des amorces, par des séquences d'ADN contigües de longueur variant approximativement de 0 à 10 kb, si les molécules totales ont une longueur variant de 0,5 à 20 kb.

Ainsi, les molécules d'ADN à analyser par amplification ne comporteront les séquences cibles en position distale que dans une fraction minoritaire. Au contraire, une contamination par un produit d'amplification antérieure apportera exclusivement un brin d'ADN cible délimité, à ses extrémités, par les séquences correspondant aux oligonucléotides amorces spécifiques et à leurs séquences complémentaires. Ces produits d'amplifications antérieures, de par leur structure, auront d'autant plus tendance à être utilisés comme substrats lors de la réaction ultérieure.

WO 89 11548 décrit un procédé de fixation de sondes sur support solide et l'utilisation de ces sondes pour la détection de produits amplifiés par PCR. Une méthode de stérilisation non spécifique de l'ADN à l'aide de DNases avant l'introduction des amorces et de l'ADN à amplifier est décrite.

EP 407291 qui fait partie de l'Etat de la Technique selon l'Art. 54(3) et (4), décrit un procédé d'amplification d'acides nucléiques comprenant un traitement de l'échantillon à amplifier par une enzyme exonucléase 5'.

L'invention vise avant tout à résoudre ce problème de contamination par des produits d'amplification antérieures, d'un milieu réactionnel convenant pour la mise en oeuvre d'un procédé PCR.

Elle vise également à optimiser les conditions d'utilisation de la PCR, en rendant cette technique aussi fiable qu'elle peut être puissante.

La Demanderesse a maintenant établi que le but visé est atteint lorsque, à la fin d'une réaction PCR antérieure, on rend les produits d'amplification obtenus inaptes à une réamplification ultérieure dans une reaction PCR ultérieure qui utilise les mêmes oligonucléotides amorces et/ou avant toute réaction PCR ultérieure faisant suite à une reaction PCR antérieure qui utilise les mêmes oligonucléotides amorces, on met en oeuvre un prétraitement prévenant sélectivement la réamplification des produits d'amplifications antérieures résultant de la réaction PCR antérieure utilisant les mêmes oligonucléotides amorces.

C'est pourquoi, l'invention a pour objet un procédé d'amplification enzymatique in vitro (procédé PCR) d'une séquence d'ADN cible présente au sein d'ADN hétérologue dans un milieu comportant une ADN polymérase et des oligonucléotides amorces, ledit procédé comportant une étape de dénaturation thermique de l'ADN, une étape d'hybridation des oligonucléotides amorces sur l'ADN, et une étape de polymérisation enzymatique par l'ADN polymérase à partir desdites amorces, lesdites étapes étant répétées de façon cyclique et l'étape de polymérisation enzymatique générant notamment des produits d'amplification de longueur finie constitués de ladite séquence d'ADN bornée à chaque extrémité par un oligonucléotide amorce ou son complément, caractérisé en ce qu'au moment désiré après l'arrêt des cycles d'amplification, on rend lesdits produits d'amplification inaptes à une réamplification ultérieure et/ou avant le début de la reaction PCR, on met en oeuvre un prétraitement prévenant sélectivement la réamplification des produits d'amplifications antérieures résultant d'une réaction PCR utilisant les mêmes oligonucléotides amorces en traitant lesdits produits d'amplifications antérieures par une quantité appropriée d'au moins une exonucléase 3'.

Ainsi, l'invention recouvre deux aspects qui peuvent être mis en oeuvre de manière cumulée ou alternative. Bien entendu, lorsque la réaction PCR est effectuée pour la première fois avec des oligonucléotides amorces donnés, il n'est pas nécessaire d'utiliser le prétraitement prévenant sélectivement la réamplification des produits d'amplification antérieures.

Pour rendre, à la fin d'une réaction PCR, les produits d'amplification inaptes a une réamplification ultérieure, on peut procéder de différentes façons, en utilisant des moyens plus ou moins destructeurs desdits produits d'amplification.

Selon l'invention, on procède à une destruction irréversible des produits d'amplification. Il peut-s'agir soit d'une destruction partielle de certaines portions du produit, suffisant à prévenir toute réamplification ultérieure.

Dans le cas où on détruit les séquences oligonucléotidiques amorces elles-mêmes, il ne reste plus dans le milieu que des molécules comportant les séquences complémentaires des oligonucléotides amorces. De telles molécules peuvent faire l'objet de multiplication arithmétique de type $x(n+1)$, mais pas d'une multiplication géométrique de type $x.2^n$ comme indiqué plus haut. Une telle réplication ne représente pas une compétition notable au regard de la réaction d'amplification complète et cette destruction partielle convient donc bien pour résoudre le problème posé par l'invention.

Pour la prévention sélective de la réamplification de produits d'amplifications antérieures éventuellement présents dans le milieu réactionnel, l'invention consiste à traiter lesdits produits d'amplifications antérieures par une quantité appropriée d'au moins une exonucléase 3'.

Les exonucléases sont des enzymes capables de dégrader l'ADN de façon séquentielle, en partant soit de l'extrémité 5', soit de l'extrémité 3'.

L'invention repose sur l'observation, déjà mentionnée, de la différence de structure et de longueur entre les produits d'amplification indésirables à ce stade, et les molécules d'ADN à analyser. Les produits d'amplifications antérieures sont caractérisés à leurs extrémités par la présence des séquences amorces ayant permis les initiations de l'amplification. En revanche, les molécules d'ADN à analyser contiennent les séquences cibles - et donc les séquences qui vont s'hybrider aux séquences amorces - réparties statistiquement le long de molécules de taille variant approximativement de 0,5 à 20 Kb. Une attaque exonucléasique d'une centaine ou de quelques centaines de bases aura donc pour effet de détruire totalement un produit d'amplification contaminant tout en préservant la grande majorité des séquences cibles pouvant se trouver dans l'échantillon à analyser.

Différents types d'exonucléases peuvent être utilisées. Dans le cas des exonucléases qui attaquent l'ADN à partir de ses extrémités 5' (exonucléases 5') le traitement supprime les séquences amorces et maintient leurs séquences complémentaires, pouvant faire l'objet uniquement de multiplications linéaires. Selon l'invention les exonucléases 3' suppriment les séquences complémentaires des amorces, supprimant ainsi de façon particulièrement avantageuse, toute possibilité d'amplification.

Suivant le type d'exonucléase utilisé, les conditions de leur introduction dans le mélange réactionnel sont plus ou moins contraignantes. En effet, certaines exonucléases ne sont pas spécifiques de l'ADN bicaténaire ou digèrent simultanément les deux brins d'ADN en partant de ses extrémités. Dans ce cas, il est préférable de contrôler de façon précise la digestion exonucléatique et de redéfinir ses paramètres pour chaque nouvelle mise en oeuvre. Ceci signifie que ses paramètres doivent à chaque fois être adaptés à la nature de l'échantillon à analyser, à la façon dont l'ADN a été extrait, à sa concentration, aux conditions de la réaction, etc.

Suivant un mode de réalisation avantageux de l'invention, on utilise des exonucléases spécifiques de l'ADN bicaténaire. Ce type d'exonucléases digère les extrémités des molécules d'ADN, ce qui n'a pas d'effet sur les molécules cibles. En effet, l'un des brins de la cible peut être détruit, mais le brin complémentaire, portant également la séquence cible, est par définition, préservé. De plus, même si le traitement exonucléasique est poursuivi de façon exhaustive, ou en présence de grandes quantités d'enzymes, la dégradation s'arrête lorsque le milieu ne contient plus de molécules d'ADN bicaténaires. A ce moment là, par définition, la moitié des séquences sont digérées, ce qui a pour effet de diviser simplement par deux le nombre de séquences cibles. Par conséquent, même si un traitement exonucléasique est poursuivi très largement au delà de ce qui est nécessaire pour neutraliser des produits d'amplification, la possibilité de détecter la séquence cible n'est nullement affectée puisque la cible est toujours présente après digestion au minimum a un nombre d'exemplaires moitié du nombre contenu dans l'échantillon de départ.

L'utilisation de ce type d'exonucléases est donc particulièrement avantageux dans la mesure où il permet d'éviter un contrôle rigoureux de la digestion exonucléasique. De plus, il permet d'effectuer le traitement exonucléasique sur la totalité du mélange réactionnel, contenant déjà les oligonucléotides amorces.

Comme exonucléases de ce type, on peut citer, de façon non exhaustive, l'exonucléase III, qui catalyse la libération de 5' mono-nucléotides à partir des extrémités 3' OH et n'est pas active sur l'ADN monocaténaire.

En pratique, il suffit de définir les conditions de la digestion exonucléasique afin d'obtenir une digestion de 20 nucléotides environ. En effet, dans ces conditions, on évite tout risque de destruction de la cible de l'amplification car même s'il n'est pas toujours possible d'obtenir une digestion rigoureusement synchrone de toutes les molécules et si certaines font l'objet de dégradations plus importantes, de l'ordre de quelques centaines de bases, cela n'a pas d'effet notable sur la capacité d'amplification des molécules cibles.

Plus précisément, le traitement par les exonucléases comporte avantageusement les étapes suivantes :
a) mélange des composants de la réaction PCR,
b) addition de l'exonucléase en quantité appropriée,
c) incubation à 37°C pendant un temps suffisant à la dégradation,
d) passage à température élevée pendant au moins 5 minutes.

Ensuite, les cycles d'amplification sont mis en oeuvre dans les conditions habituelles. L'étape d) s'effectue de préférence à une température supérieure à 90°C : elle permet à la fois la dénaturation de l'ADN pour initier les cycles d'amplification et la dégradation irréversible de l'activité exonucléasique.

L'invention a également pour objet une trousse pour la mise en oeuvre d'un procédé PCR comportant une étape de prétraitement des échantillons à analyser par une exonucléase, qui comprend une quantité appropriée d'au moins une exonucléase.

Bien entendu, il est également possible d'utiliser la digestion exonucléasique, dans des conditions très ménagées, comme traitement final à la fin d'une réaction PCR. Ce traitement ménagé a pour effet de détruire les séquences de reconnaissance des amorces tout en conservant les séquences amplifiées. Toutefois, à ce stade, un contrôle beaucoup plus fin des conditions de la digestion exonucléasique est nécessaire pour respecter l'intégrité des séquences internes des produits d'amplification.

D'une façon générale, toutes les variantes envisageables conformément à l'invention peuvent être mises en oeuvre de façon séparée ou combinée, en ce qui concerne aussi bien les différentes variantes de procédé que les différents types d'oligonucléotides amorces que l'on peut préparer.

Les exemples suivants illustrent l'invention de façon non limitative.

Exemple 1

Etude des effets de la digestion par l'exonucléase III sur des produits d'amplification contaminants

Dans une première expérience, on effectue une cinétique de digestion par de l'exonucléase III sur un mélange de deux fragments d'ADN longs de 5000 et de 300 paires de bases et l'on suit les effets de la digestion par analyse sur gel et coloration au bromure d'éthidium. La digestion est effectuée dans le tampon utilisé usuellement pour effectuer les amplifications.

On voit que le fragment de 300 pdb disparait au bout de 5 minutes de digestion mais que le fragment de 5000 pdb voit tout d'abord sa taille décroître entre 5 et 10 minutes de digestion pour être ensuite converti de façon stable en ADN monocaténaire à partir de 20 minutes. Le produit de digestion obtenu à ce moment n'est pas modifié par une incubation plus prolongée avec l'enzyme.

Cette expérience montre qu'une incubation prolongée d'ADN en présence d'exonucléase III dans le tampon d'amplification ne dégrade pas ces molécules d'ADN mais aboutit à un équilibre où l'ADN monocaténaire est stable même pendant des incubations longues. Elle confirme également que cette digestion déstabilise plus rapidement les courtes molécules (comme le sont les produits d'amplification) que les longues (comme le sont les molécules d'ADN cible).

Dans une seconde expérience, on effectue une amplification sur de l'ADN génomique humain en utilisant les amorces PCO2, Nature, (1986) 324, p.163-166 et PCO4, Science, (1985) 280, p. 1350-1354 qui permettent d'amplifier un fragment du gène globine et l'on isole la bande ainsi produite qui est utilisée comme agent de contamination artificiel.

On prépare alors deux séries de tubes, l'un comportant 1 $\mu$g d'ADN humain et l'autre 1 $\mu$g d'ADN plasmidique linéaire ne contenant pas de séquences amplifiables par les amorces utilisées plus 0,01 pg d'ADN contaminant. Ces deux séries sont traitées par 10 u d'exonucléase III pendant 0, 5, 10, 20, 40 et 60 mn puis soumises à amplification dans des conditions usuelles.

La détection des produits d'amplification s'effectue par électrophorèse directe sur gel d'agarose 1,4 % et coloration au bromure d'éthidium.

Les résultats de cette expérience sont les suivants :

| | Présence du produit amplifié | | | | | |
|---|---|---|---|---|---|---|
| Digestion par l'exonucléase III | 0 | 5 | 10 | 20 | 40 | 60 (minutes) |
| ADN génomique | + | + | + | + | + | + |
| ADN contaminant | + | - | - | - | - | - |

Ces résultats montrent 1) qu'une digestion même poussée d'ADN génomique par de l'exonucléase III ne modifie pas le potentiel amplifiable d'une cible donnée, unique, au sein de cet ADN mais que 2) le même traitement appliqué à un produit amplifié, purifié (donc équivalent à un contaminant potentiel) et introduit comme contaminant artificiel dans le milieu, détruit sa capacité à être amplifié après les premières minutes de digestion.

**Revendications**

1. Procédé d'amplification enzymatique in vitro d'une séquence d'ADN cible présente au sein d'ADN hétérologue dans un milieu comportant une ADN polymérase et des oligonucléotides amorces, ledit procédé comportant une étape de dénaturation thermique de l'ADN, une étape d'hybridation des oligonucléotides amorces sur l'ADN, et une étape de polymérisation enzymatique par l'ADN polymérase à partir desdites amorces, lesdites étapes étant répétées de façon cyclique et l'étape de polymérisation enzymatique générant notamment des produits d'amplification de longueur finie constitués de ladite séquence d'ADN bornée à chaque extrémité par un oligonucléotide amorce ou son complément, caractérisé en ce qu'au moment désiré après l'arrêt des cycles d'amplification, on rend lesdits produits d'amplification inaptes à une réamplification ultérieure et/ou avant le début de la réaction PCR, on met

# EP 0 536 214 B1

en oeuvre un prétraitement prévenant sélectivement la réamplification de produit d'amplifications antérieures résultant d'une réaction PCR utilisant les mêmes oligonucléotides amorces en traitant lesdits produits d'amplifications antérieures par une quantité appropriée d'au moins une exonucléase 3'.

2. Procédé selon la revendication 1, caractérisé en ce qu'on utilise une exonucléase spécifique de l'ADN bicaténaire.

3. Procédé selon la revendication 2, caractérisé en ce qu'on utilise l'exonucléase III.

4. Procédé selon l'une des revendications 1 à 3, caractérisé en ce qu'il comporte, avant le début de la réaction PCR, les étapes suivantes:
   a) mélange des composants de la réaction PCR,
   b) addition de l'exonucléase 3' en quantité appropriée,
   c) incubation à 37°C pendant un temps suffisant à la dégradation,
   d) passage à température élevée pendant au moins 5 minutes.

5. Trousse pour la mise en oeuvre d'un procédé PCR selon l'une des revendications 1 à 4, caractérisée en ce qu'elle comprend une quantité appropriée d'au moins une exonucléase 3'.

## Claims

1. Method for the in vitro enzymatic amplification of a target DNA sequence present within heterologous DNA in a medium containing a DNA polymerase and oligonucleotide primers, the said method entailing a step of thermal denaturation of the DNA, a step of hybridisation of the oligonucleotide primers with the DNA and a step of enzymatic polymerisation with the DNA polymerase from the said primers, the said steps being repeated cyclically and the step of enzymatic polymerisation generating, in particular, amplification products of finite length consisting of the said DNA sequence flanked at each end by an oligonucleotide primer or its complement, characterised in that, at the desired time after stopping the amplification cycles, the said amplification products are rendered incapable of a subsequent reamplification, and/or, before the beginning of the PCR reaction, a pretreatment is carried out which selectively prevents reamplification of products of previous amplifications resulting from a PCR reaction in which the same oligonucleotide primers are used, by treating the said products of previous amplifications with a suitable quantity of at least one 3' exonuclease.

2. Method according to Claim 1, characterised in that an exonuclease specific for double-stranded DNA is used.

3. Method according to Claim 2, characterised in that exonuclease III is used.

4. Method according to one of Claims 1 to 3, characterised in that it comprises, before the beginning of the PCR reaction, the following steps:
   a) mixing of the components of the PCR reaction,
   b) addition of a suitable quantity of the 3' exonuclease,
   c) incubation at 37°C for sufficient time for the degradation,
   d) raising to a high temperature for at least 5 minutes.

5. Kit for carrying out a PCR method according to one of Claims 1 to 4, characterised in that it comprises a suitable quantity of at least one 3' exonuclease.

## Patentansprüche

1. Verfahren zur enzymatischen in vitro-Amplifikation einer DNA-Ziel-Sequenz, die im Innern einer heterologen DNA in einem Medium vorliegt, das eine DNA-Polymerase und Starter-Oligonucleotide aufweist, wobei das genannte Verfahren umfaßt eine Stufe zur thermischen Denaturierung der DNA, eine Stufe zur Hybridisierung der Starter-Oligonucleotide auf der DNA und eine Stufe zur enzymatischen Polymerisation der genannten Starter-Oligonucleotide durch die DNA-Polymerase, wobei die genannten Stufen cyclisch wiederholt werden und die Stufe der enzymatischen Polymerisation insbesondere Amplifikations-Produkte mit einer begrenzten Länge ergibt, die bestehen aus der genannten DNA-Sequenz, die

8

an jedem Ende durch ein Starter-Oligonucleotid oder sein Komplement begrenzt ist, dadurch gekennzeichnet, daß man im gewünschten Augenblick nach Beendigung der Amplifikations-Cyclen die genannten Amplifikations-Produkte unfähig macht für eine Schluß-Reamplifikation und/oder vor Beginn der PCR-Reaktion eine Vorbehandlung durchführt, welche die Reamplifikation der vorhergehenden Amplifikations-Produkte, die aus einer PCR-Reaktion resultieren, selektiv verhindert durch Verwendung der gleichen Starter-Oligonucleotide zur Behandlung der genannten vorhergehenden Amplifikations-Produkte mit einer geeigneten Menge mindestens einer 3'-Exonuclease.

2. Verfahren nach Anspruch 1, dadurch gekennzeichnet, daß man eine für die Doppelstrang-DNA spezifische Exonuclease verwendet.

3. Verfahren nach Anspruch 2, dadurch gekennzeichnet, daß man die Exonuclease III verwendet.

4. Verfahren nach einem der Ansprüche 1 bis 3, dadurch gekennzeichnet, daß es vor Beginn der PCR-Reaktion die folgenden Stufen umfaßt:
   a) Mischen der Komponenten der PCR-Reaktion,
   b) Zugabe der 3'-Exonuclease in einer geeigneten Menge,
   c) Inkubation bei 37°C für eine für den Abbau ausreichende Zeitspanne und
   d) Erhitzen auf eine erhöhte Temperatur während mindestens 5 min.

5. Set für die Durchführung eines PCR-Verfahrens nach einem der Ansprüche 1 bis 4, dadurch gekennzeichnet, daß es eine geeignete Menge mindestens einer 3'-Exonuclease umfaßt.

Cycle 1

Cycle 2

Cycle 3

## FIG-1